# EUROPÄISCHE PATENTSCHRIFT

(11) **EP 0 821 951 B1**
(45) Veröffentlichungstag und Bekanntmachung des Hinweises auf die Patenterteilung: **02.02.2000**
(21) Anmeldenummer: 97112089.4
(22) Anmeldetag: 15.07.1997
(51) Int. Cl.: A61K 31/215, A61K 31/225, A61K 33/10

(54) **Verfahren zur Herstellung einer Bicarbonat enthaltenden Infusions- oder Dialysierlösung**
Process for preparing a bicarbonate-containing infusion- or dialysis solution
Procédé pour la fabrication d'une solution d'infusion ou de dialyse contenant des bicarbonates

(30) Priorität: 01.08.1996 DE 19631124
(43) Veröffentlichungstag der Anmeldung: 04.02.1998
(73) Patentinhaber: Fresenius Medical Care Deutschland GmbH, 61350 Bad Homburg v.d.H. (DE)
(72) Erfinder: Knerr, Thomas, Dr., 66606 St. Wendel (DE); Wild, Thomas, Dr., 66571 Eppeldorn (DE)
(74) Vertreter: Laufhütte, Dieter, Dr.-Ing.

(56) Entgegenhaltungen:
- EP-A- 0 399 549
- EP-A- 0 437 274
- EP-A- 0 490 307
- WO-A-96/01118
- FR-A- 2 467 599

## Beschreibung

Die Erfindung betrifft ein Verfahren zur Herstellung einer Bicarbonat enthaltenden Infusions- oder Dialysierlösung mit einem physiologisch verträglichen pH-Wert, eine Verwendung dieses Verfahrens zur Herstellung einer Peritonealdialyse-Lösung und eine Infusions- oder Dialysierlösung, vorzugsweise eine Peritonealdialyse-Lösung. Beispielsweise bei Patienten mit akuter oder chronischer Niereninsuffizienz muß die eingeschränkte Nierenfunktion durch alternative Verfahren kompensiert werden. Derartige alternative Verfahren sind die Hämodialyse und die Peritonealdialyse. Bei der sogenannten kontinuierlichen, ambulanten Peritonealdialyse (CAPD) wird die Peritonealhöhle von nierenkranken Patienten mehrfach täglich mit einer frischen Peritonealdialyse-Lösung gefüllt. Bei dieser Art der Dialyse findet die Entgiftung und Entwässerung über die den gesamten Bauchraum auskleidende Peritonealmembran statt. Das Peritoneum bildet beim Stoffaustausch eine semipermeable Membran, durch die gelöste Stoffe im Sinne der Diffusion durchtreten. Innerhalb von zwei bis drei Stunden kommt es durch Diffusion zu einem Konzentrationsanstieg der harnpflichtigen Substanzen in der frisch eingefüllten Peritonealdialyselösung.

Gleichzeitig erfolgt entsprechend dem osmotischen Gleichgewicht der Flüssigkeitsentzug durch Ultrafiltration. Die Peritonealdialyse-Lösung verbleibt über 4 bis 8 Stunden im Bauchraum und wird danach durch einen Katheter nach außen abgelassen. Die Prozedur erfolgt in der Regel viermal täglich, d.h. abgesehen von den Zeiten des Ein- und Auslaufs ist die Peritonealhöhle ständig mit Lösung gefüllt.

Neben Elektrolyten und der oder den osmotisch wirksamen Substanzen enthalten CAPD-Lösungen eine Puffersubstanz, die den Säure-Base-Haushalt des Patienten reguliert, insbesondere eine Acidosis verhindern oder beheben soll. Zur Pufferung wird bislang im wesentlichen Natriumlactat eingesetzt. In Untersuchungen zur Biokompatibilität von CAPD-Lösungen zeigte sich jedoch, daß lactathaltige Lösungen negativ auf die Lebensfunktion menschlicher Zellen wirken (vgl. z.B. Bronswijk, Verbrugh, Bos, Heezius, Oe, van der Meulen, Verhoef, Cytotoxic effects of commercial continuous ambulatory peritoneal dialysis (CAPD) fluids, Peritoneal Dialysis International 9 (1989) S. 197-202 oder Witowski, Topley, Jörres, Effect of lactate-buffered peritoneal dialysis fluids on human peritoneal mesothelial cell interleukin-6 and prostaglandin synthesis, Kidney International 46 (1994): 282-293).Als verträglicher haben sich bicarbonathaltige CAPD-Lösungen mit physiologischem pH-Wert erwiesen (vgl. z.B. Schambye, Pederson, Christensen, Berthelsen, Wang, The cytotoxicity of CAPD solutions with different bicarbonate/lactate ratios, Peritoneal Dialysis International 13, Suppl. 2 (1993): S. 116-118).

Aus EP 0 399 549 ist eine CAPD-Lösung bekannt, die durch Mischen zweier Kompartimente hergestellt wird, deren eines Bicarbonationen enthält. Die resultierende Mischlösung hat einen physiologischen pH-Wert im Bereich von 7,2 bis 7,4 und enthält 20 bis 40 mmol/l Bicarbonationen. Die Herstellung der bicarbonathaltigen Lösung ist schwierig, da sich nach Lösen des Salzes stets Kohlendioxid im Gleichgewicht gemäß der folgenden Formel bildet: 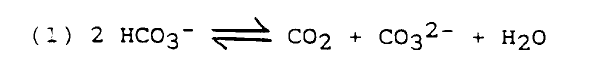 Kohlendioxid kann die Lösung beim Ansetzen, Rühren und Lagern verlassen, was zu einem Anstieg des pH-Werts führt. Um den pH-Wert innerhalb des angestrebten pH-Wert-Bereichs zu halten, muß die Lösung vor der Abfüllung regelmäßig mit Kohlendioxid oder einer anderen Säure versetzt werden. Im letzteren Fall sinkt jedoch der Gehalt an Bicarbonat, was problematisch in Hinblick auf den deklarierten Gehalt und somit in Hinblick auf die Verwendbarkeit der Lösung zur CAPD ist.

CAPD-Lösungen werden zumeist in flexible Kunststoffbehälter gefüllt, die anschließend einer Heißdampfsterilisation unterzogen werden. Obwohl Folien mit einer Sperrwirkung gegenüber CO₂ erhältlich sind, kommt es bei diesem Prozeß unvermeidlich zu einem Verlust an CO₂ und einem Anstieg des pH-Werts.

Eine weitere Schwierigkeit bei der CAPD-Lösung besteht darin, daß bei einem zu hohen pH-Wert Carbonat entsteht, welches mit den in der Lösung befindlichen Elektrolyten, insbesondere mit Calciumionen, einen schwer löslichen Niederschlag, beispielsweise in Form einer Calciumcarbonat-Ausfällung bildet. Für die Stabilität der Lösung ist daher die Einstellung des pH-Wertes von entscheidender Bedeutung.

Aus der EP 564 672 A1 ist eine Peritonealdialyse-Lösung mit physiologischer Zusammensetzung im Bezug auf den pH-Wert bekannt, bei der die Stabilität des pH-Werts verbessert ist. Diese Peritonealdialyse-Lösung wird unmittelbar vor Gebrauch aus zwei Einzel-Lösungen erhalten, wobei die erste Einzel-Lösung eine osmotisch wirksame Substanz und die zweite Einzel-Lösung Bicarbonationen enthält. Die erste Einzel-Lösung enthält Anionen von Mono- und/oder Dicarbonsäuren und besitzt einen pH-Wert von 4,5 bis 5,8 und die zweite Einzel-Lösung enthält eine Aminosäure-Komponente oder eine Peptid-Komponente und besitzt einen pH-Wert von 7,2 bis 10,0. Die gebrauchsfertige Lösung enthält 23 bis 26 mmol/l Bicarbonationen. Bei dieser vorbekannten Peritonealdialyse-Lösung ist es nach wie vor notwendig, die endgültig eingesetzte Lösung aus den getrennt bereitzustellenden Einzel-Lösungen zusammenzumischen und dann innerhalb kurzer Zeit zu verbrauchen.

Die zuvor bezogen auf eine Peritonealdialyse angestellten Betrachtungen lassen sich allgemein auf eine Bicarbonat enthaltende Infusions- oder Dialysierlösung übertragen.

EP 437 274 beschreibt eine Bicarbonat- und Calciumionen enthaltende Infusions- und Dialysierlösung, deren CO₂- Partialdruck durch ein Puffergemisch konstant gehalten wird. Aus dem Puffergemisch wird kontinuierlich CO₂ nachgeliefert. In diesem Dokument gibt es keine Angabe daß CO₂ auch während der Hitzesterilisation aus der Hydrolyse eines Carbonsäure-Ester geliefert werden kann.

Allen Verfahren ist es gemeinsam, daß im Rahmen der vorzusehenden Heißdampfsterilisation bzw. Hitzesterilisation CO₂ entweicht und der pH-Wert dadurch ansteigt.

Aufgabe der Erfindung ist es daher, ein Verfahren zur Herstellung einer Bicarbonat enthaltenden Infusions- oder Dialysierlösung mit einem physiologisch verträglichen pH-Wert zu entwickeln, bei dem der entsprechend eingestellte pH-Wert möglichst geringen Schwankungen unterliegt.

Erfindungsgemäß wird diese Aufgabe ausgehend von einem gattungsgemäßen Verfahren durch die kennzeichnenden Merkmale des Anspruchs 1 gelöst. Demnach wird in der Lösung Carbonsäureester vorgelegt und die Lösung wird einer Hitzebehandlung über 100°C unterworfen, so daß das Carbonsäureester nahezu vollständig in Carbonsäure und Alkohol zersetzt wird.

In der Lösung werden erfindungsgemäß mindestens 2 mmol/l mindestens eines Carbonsäureesters vorgelegt.

Die eingesetzten Carbonsäureester sind neutrale Derivate von Carbonsäuren. Wasserlösliche Carbonsäureester werden in Abhängigkeit vom pH-Wert der Lösung und der Temperatur unterschiedlich schnell entsprechend der Gleichung 2 hydrolysiert.

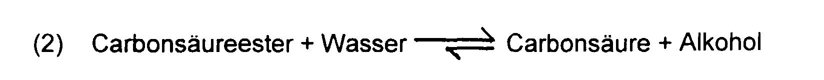

Bei erhöhten Temperaturen verläuft die Hydrolyse schnell und quantitativ. Unterzieht man die Lösung einer Hitzebehandlung über 100°C, wie dies bei der Heißdampfsterilisation erfolgt, kann man von einer annähernd quantitativen Hydrolysierung des Carbonsäureesters ausgehen. Findet die Hydrolyse des Carbonsäureesters in Gegenwart einer Base statt, so bildet sich aus der nach Gleichung 2 entstandenen Carbonsäure das entsprechende Salz. Ist die Base beispielsweise Natriumbicarbonat, so entsteht nach Gleichung (3) das Natriumsalz der Carbonsäure und Kohlendioxid. Bei der Hydrolyse des Esters kommt es also zu einer Ansäuerung der Lösung.

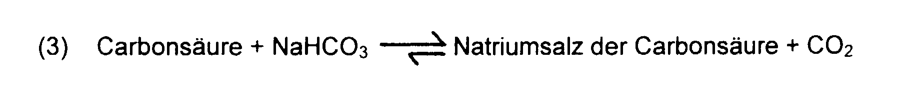

Durch die vorgegebene Reaktion läßt sich erfindungsgemäß der pH-Wert der bicarbonathaltigen Lösung verläßlich einstellen.

Vorteilhafte Ausgestaltungen des erfindungsgemäßen Verfahrens ergeben sich aus den sich an den Hauptanspruch anschließenden Unteransprüchen.

Besonders vorteilhaft ist es, wenn in der Lösung mindestens 2 mmol/l, bevorzugt 2 bis 5 mmol/l, besonders bevorzugt 3 mmol/l Carbonsäureester vorgelegt werden.

Gemäß einer bevorzugten Ausgestaltung der vorliegenden Erfindung ist vorgesehen, daß die Carbonsäure aus der Gruppe Essigsäure, Propansäure, Milchsäure, Brenztraubensäure, 4-Hydroxybuttersäure, Bernsteinsäure, Maleinsäure, Fumarsäure, Äpfelsäure, Oxalessigsäure, Glutarsäure, 2-Oxoglutarsäure, Zitronensäure, Isozitronensäure und Glukonsäure ausgewählt ist.

Der Carbonsäureester kann ein Ester eines ein- oder mehrwertigen Alkohols sein. Besonders vorteilhaft ist es, wenn der Carbonsäureester ein Ester des Ethanols, Propanols, Isopropanols oder Glyzerins oder ein zyklischer Ester (Lacton) oder ein Kohlensäureester ist.

In weiterer Ausgestaltung der Erfindung ist vorgesehen, daß das erfindungsgemäße Verfahren zur Herstellung einer Lösung zur Peritonealdialyse angewendet wird, wobei die Lösung zusätzlich, wie im Stand der Technik bekannt, ein Osmotikum, beispielsweise Glukose, enthält.

Die Erfindung betrifft ferner eine medizinische Infusions- oder Dialyselösung, vorzugsweise eine Peritonealdialyse-Lösung, die mindestens die Kombination folgender Komponenten enthält: Bicarbonat, vorzugsweise Natriumbicarbonat, eine physiologisch verträgliche Carbonsäure und einen physiologisch verträglichen Alkohol, wobei ein pH-Wert im Bereich von 6,9 bis 7,8 eingestellt ist und wobei die Carbonsäure sowie der Alkohol durch die Hydrolyse eines Carbonsäureesters gebildet wurden. Bevorzugte Ausführungsformen dieser medizinischen Infusions- oder Dialyselösung nach Anspruch 12 ergeben sich aus den Unteransprüchen 13 bis 17.

Besonders vorteilhaft ist es, wenn mindestens 2 mmol/l, vorzugsweise 2 bis 10 mmol/l Carbonsäureester vorgelegt werden.

In weiterer Ausgestaltung der vorliegenden Erfindung ist vorgesehen, daß die Carbonsäure aus der Gruppe Essigsäure, Propansäure, Milchsäure, Brenztraubensäure, 4-Hydroxybuttersäure, Bernsteinsäure, Maleinsäure, Fumarsäure, Äpfelsäure, Oxalessigsäure, Glutarsäure, 2-Oxoglutarsäure, Zitronensäure, Isozitronensäure und Glukonsäure ausgewählt wurde.

Besonders vorteilhaft ist es, wenn die erfindungsgemäße Lösung unmittelbar vor ihrer Anwendung durch Mischung zweier Lösungen erhalten wird, von denen eine Calcium-lonen und die andere Natriumbicarbonat enthält.

Die erfindungsgemäße Lösung kann durch Mischen zweier Lösungen erhalten werden, von denen eine mindestens eine osmotisch wirksame Substanz, vorzugsweise Glukose, und die andere Bicarbonat enthält.

Weitere Einzelheiten und Vorteile der Erfindung ergeben sich aus den im folgenden näher beschriebenen Beispielen:

In einem Vergleichsbeispiel sind 10 mmol/l Ethyllactat in eine Lösung von 60 mmol/l Natriumbicarbonat während 24 Stunden bei Raumtemperatur auf den Hydrolysegrad des Esters nach folgender Gleichung (Gleichung 4) untersucht worden:

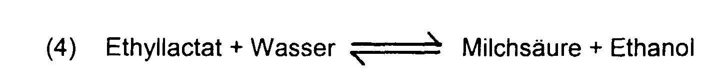

Bei Raumtemperatur wurden hier während 24 Stunden nur ca. 6% des Esters hydrolysiert.

Im Vergleich hierzu wurde die vorgenannte Lösung auf 121°C erhitzt. Dies entspricht den Bedingungen einer Heißdampfsterilisation. Das Ergebnis ist in der Tabelle 1 wiedergegeben.

**Tabelle 1:**

| Sterilisationszeit | Ausgangswert [ppm] | Ethyllactat [ppm] | Hydrolysegrad |
|---|---|---|---|
| 5 min | 1181 | 287 | 76 % |
| 10 min | 1181 | 107 | 91 % |
| 20 min | 1181 | 2.7 | 99,8 % |
| 30 min | 1181 | <1 | >99,9 % |

Aus der Tabelle 1 wird deutlich, daß nach ca. 20 Minuten der Ester annähernd quantitativ hydrolysiert ist.

Bei dem hier genannten Beispiel wird sich neben der Milchsäure in Anwesenheit von Natriumbicarbonat auch das entsprechende Salz der Carbonsäure, Natriumlactat, bilden. Gemäß Gleichung 5 ergibt sich

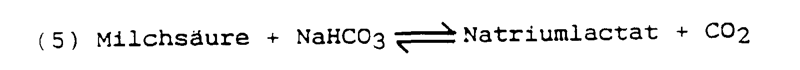

In der Tabelle 2 sind beispielhaft pH-Werte aufgeführt, die beim Einsatz verschiedener Carbonsäureester in verschiedenen Konzentrationen in der bicarbonathaltigen Lösung enthalten werden. Diese Beispiele sind nicht als Einschränkung auf die Verwendung bestimmter Carbonsäureester oder bestimmter Konzentrationen dieser Ester im Rahmen der vorliegenden Erfindung zu verstehen.

Alternativ zu Carbonsäureester können auch Kohlensäureester eingesetzt werden, die bei der Verseifung zu H₂CO₃ bzw. Wasser und Kohlendioxid werden.

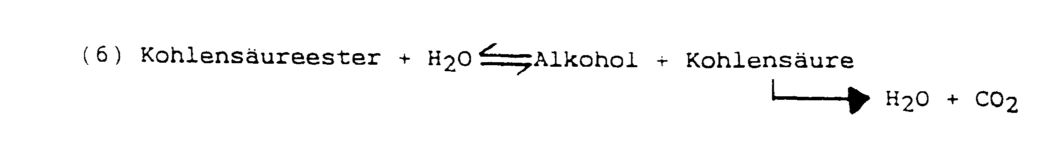

Im Sinne der Erfindung ist Kohlensäureester unter Carbonsäure-ester zu subsumieren bzw. Kohlensäure unter Carbonsäuren.

Die dem in Tabelle 2 wiedergegebenen Versuchsergebnis zugrundeliegenden Lösungen wurden in 11-Kunststoffbeutel abgefüllt. Die gefüllten Beutel wurden bei einer Temperatur von 121°C autoklaviert. Nach Erreichen der Autoklavtemperatur wurde diese für 30 Minuten aufrechterhalten. Die sich nach der Sterilisation ergebenden pH-Werte sind in der Tabelle 2 angegeben.

**Tabelle 2**

| Versuchs-Nr. | NaHCO₃-Konzentration | Art und Konzentration des Carbonsäureesters | pH-Wert nach Sterilisation |
|---|---|---|---|
| 1 | 50 mmol/l | Glucono-δ-lacton 5 mmol/l | 7,76 |
| 2 | 50 mmol/l | Glucon-δ-lacton 10 mmol/l | 7,23 |
| 3 | 50 mmol/l | Diethylsuccinat 3 mmol/l | 7,50 |
| 4 | 50 mmol/l | Diethylsuccinat 5 mmol/l | 7,25 |
| 5 | 55 mmol/l | Diethyltartrat 5 mmol/l | 7,22 |
| 6 | 55 mmol/l | Triethylcitrat 3,5 mmol/l | 7,50 |
| 7 | 55 mmol/l | Ethyllactat 10 mmol/l | 7,20 |
| 8 | 55 mmol/l | Diethylcarbonat 5 mmol/l | 7,70 |
| 9 | 60 mmol/l | Ethyllactat 10 mmol/l | 7,20 |

## Patentansprüche

1. Verfahren zur Herstellung einer Bicarbonat und physiologisch verträgliche Carbonsäure enthaltenden Infusions- oder Dialysierlösung zum Einstellen eines physiologisch verträglichen pH-Wertes,
**dadurch gekennzeichnet**,
daß in der Lösung mindestens 2 mmol/l mindestens eines Carbonsäure-Esters vorgelegt wird und daß die Lösung einer Hitzebehandlung über 100°C unterworfen wird, so daß der Carbonsäure-Ester nahezu vollständig in Carbonsäure und Alkohol zersetzt wird.

2. Verfahren nach Anspruch 1, dadurch gekennzeichnet, daß in der Lösung mindestens 2 mmol/l, bevorzugt 2 bis 5 mmol/l, besonders bevorzugt 3 mmol/l, Carbonsäure-Ester vorgelegt werden.

3. Verfahren nach Anspruch 1 oder Anspruch 2, dadurch gekennzeichnet, daß die Lösung auf 121°C erhitzt wird.

4. Verfahren nach einem der Ansprüche 1 bis 3, dadurch gekennzeichnet, daß die Hitzebehandlung im Wege einer Heißdampfsterilisation durchgeführt wird.

5. Verfahren nach einem der Ansprüche 1 bis 4, dadurch gekennzeichnet, daß die Carbonsäure aus der Gruppe Essigsäure, Propansäure, Milchsäure, Brenztraubensäure, 4-Hydroxybuttersäure, Bernsteinsäure, Maleinsäure, Fumarsäure, Äpfelsäure, Oxalessigsäure, Glutarsäure, 2-Oxoglutarsäure, Zitronensäure, Isozitronensäure und Glukonsäure stammt.

6. Verfahren nach einem der Ansprüche 1 bis 5, dadurch gekennzeichnet, daß der Carbonsäure-Ester ein Ester eines ein- oder mehrwertigen Alkohols ist.

7. Verfahren nach Anspruch 6, dadurch gekennzeichnet, daß der Carbonsäure-Ester ein Ester des Ethanols, Propanols, Isopropanols oder Glyzerins oder ein zyklischer Ester (Lakton) oder ein Kohlensäureester ist.

8. Verfahren nach einem der Ansprüche 1 bis 7, dadurch gekennzeichnet, daß der Carbonsäure-Ester Ethyllaktat ist.

9. Verfahren nach einem der vorangehenden Ansprüche, dadurch gekennzeichnet, daß das Bicarbonat Natriumbicarbonat ist.

10. Verfahren nach einem der vorangehenden Ansprüche, dadurch gekennzeichnet, daß das Verfahren zur Herstellung einer Lösung zur Peritonealdialyse angewendet wird, wobei die Lösung zusätzlich ein Osmotikum enthält.

11. Verfahren nach Anspruch 10, dadurch gekennzeichnet, daß als Osmotikum Glucose in der Lösung enthalten ist.

12. Medizinische Infusions- oder Dialyselösung, vorzugsweise Peritonealdialyselösung, mit einem pH-Wert im Bereich von 6,9 bis 7,8, mit mindestens folgenden Komponenten: Bicarbonat, vorzugsweise Natriumbicarbonat, eine physiologisch verträgliche Carbonsäure und einen physiologisch verträglichen Alkohol, wobei die Carbonsäure sowie der Alkohol durch die Hydrolyse eines Carbonsäure-Esters gebildet wurden.

13. Lösung nach Anspruch 12, dadurch gekennzeichnet, daß mindestens 2 mmol/l, vorzugsweise 2 bis 10 mmol/l Carbonsäure-Ester vorgelegt werden.

14. Lösung nach Anspruch 12 oder 13, dadurch gekennzeichnet, daß die Carbonsäure aus der Gruppe Essigsäure, Propansäure, Milchsäure, Brenztraubensäure, 4-Hydroxybuttersäure, Bernsteinsäure, Maleinsäure, Fumarsäure, Äpfelsäure, Oxalessigsäure, Glutarsäure, 2-Oxoglutarsäure, Zitronensäure, Isozitronensäure und Glukonsäure stammt.

15. Lösung nach einem der Ansprüche 12 bis 14, dadurch gekennzeichnet, daß der Carbonsäure-Ester Ethyllaktat ist.

16. Lösung nach einem der Ansprüche 12 bis 15, dadurch gekennzeichnet, daß zusätzlich eine osmotisch wirksame Substanz, vorzugsweise Glukose, enthalten ist.

17. Lösung nach einem der Ansprüche 12 bis 16, dadurch gekennzeichnet, daß sie unmittelbar vor ihrer Anwendung durch Mischung zweier Lösungen erhalten wird, von denen eine Calcium-lonen enthält und die andere Natriumbicarbonat.

18. Lösung nach einem der vorangehenden Ansprüche, dadurch gekennzeichnet, daß sie durch Mischen zweier Lösungen, von denen eine mindestens eine osmotisch wirksame Substanz, vorzugsweise Glucose, und die andere Bicarbonat enthält, erhalten wird.

## Claims

1. Process for the preparation of an infusion or dialysis solution containing bicarbonate and physiologically tolerated carboxylic acid for adjusting a physiologically tolerated pH, characterized in that at least 2 mmol/l of at least one carboxylic ester is introduced into the solution, and in that the solution is subjected to a heat treatment at above 100°C so that the carboxylic ester is decomposed virtually completely into carboxylic acid and alcohol.

2. Process according to Claim 1, characterized in that at least 2 mmol/l, preferably 2 to 5 mmol/l, particularly preferably 3 mmol/l, of carboxylic ester are introduced into the solution.

3. Process according to Claim 1 or Claim 2, characterized in that the solution is heated to 121°C.

4. Process according to any of Claims 1 to 3, characterized in that the heat treatment is carried out by means of superheated steam sterilization.

5. Process according to any of Claims 1 to 4, characterized in that the carboxylic acid is derived from the group of acetic acid, propanoic acid, lactic acid, pyruvic acid, 4-hydroxybutyric acid, succinic acid, maleic acid, fumaric acid, malic acid, oxaloacetic acid, glutaric acid, 2-oxoglutaric acid, citric acid, isocitric acid and gluconic acid.

6. Process according to any of Claims 1 to 5, characterized in that the carboxylic ester is an ester of a monohydric or polyhydric alcohol.

7. Process according to Claim 6, characterized in that the carboxylic ester is an ester of ethanol, propanol, isopropanol or glycerol or a cyclic ester (lactone) or a carbonic ester.

8. Process according to any of Claims 1 to 7, characterized in that the carboxylic ester is ethyl lactate.

9. Process according to any of the preceding claims, characterized in that the bicarbonate is sodium bicarbonate.

10. Process according to any of the preceding claims, characterized in that the process is used to prepare a solution for peritoneal dialysis, in which case the solution additionally contains an osmotic agent.

11. Process according to Claim 10, characterized in that glucose is present as osmotic agent in the solution.

12. Medical infusion or dialysis solution, preferably peritoneal dialysis solution, having a pH in the range from 6.9 to 7.8, and having at least the following components: bicarbonate, preferably sodium bicarbonate, a physiologically tolerated carboxylic acid and a physiologically tolerated alcohol, where the carboxylic acid and the alcohol have been formed by hydrolysis of a carboxylic ester.

13. Solution according to Claim 12, characterized in that at least 2 mmol/l, preferably 2 to 10 mmol/l, of carboxylic ester are introduced.

14. Solution according to Claim 12 or 13, characterized in that the carboxylic acid is derived from the group of acetic acid, propanoic acid, lactic acid, pyruvic acid, 4-hydroxybutyric acid, succinic acid, maleic acid, fumaric acid, malic acid, oxaloacetic acid, glutaric acid, 2-oxoglutaric acid, citric acid, isocitric acid and gluconic acid.

15. Solution according to any of Claims 12 to 14, characterized in that the carboxylic ester is ethyl lactate.

16. Solution according to any of Claims 12 to 15, characterized in that an osmotically active substance, preferably glucose, is additionally present.

17. Solution according to any of Claims 12 to 16, characterized in that it is obtained immediately before its use by mixing two solutions, one of which contains calcium ions and the other sodium bicarbonate.

18. Solution according to any of the preceding claims, characterized in that it is obtained by mixing two solutions, one of which contains at least one osmotically active substance, preferably glucose, and the other contains bicarbonate.

## Revendications

1. Procédé pour la fabrication d'une solution de perfusion ou de dialyse contenant un bicarbonate et de l'acide carboxylique physiologiquement supportable pour l'ajustement d'une valeur de pH physiologiquement supportable,
caractérisé en ce que
il y a, dans la solution, au moins 2 mmoles/l d'au moins un ester d'acide carboxylique et en ce que la solution est soumise à un traitement à la chaleur au-delà de 100°C de façon que l'ester d'acide carboxylique soit presque totalement transformé en acide carboxylique et alcool.

2. Procédé selon la revendication 1, caractérisé en ce que dans la solution, il y a au moins 2 mmoles/l, préférentiellement 2 à 5 mmoles et particulièrement préférentiellement 3 mmoles/l d'ester d'acide carboxylique.

3. Procédé selon la revendication 1 ou la revendication 2, caractérisé en ce que la solution est chauffée à 121°C.

4. Procédé selon l'une des revendications 1 à 3, caractérisé en ce que le traitement à la chaleur est accompli sur le parcours d'une stérilisation à la vapeur surchauffée.

5. Procédé selon l'une des revendications 1 à 4, caractérisé en ce que l'acide carboxylique provient du groupe acide acétique, acide propanoïque, acide lactique, acide pyruvique, acide 4-hydroxybutyrique, acide succinique, acide maléïque, acide fumarique, acide malique, acide oxalique, acide glutarique, acide 2-oxoglutarique, acide citrique, acide isocitrique et acide gluconique.

6. Procédé selon l'une des revendications 1 à 6, caractérisé en ce que l'ester d'acide carboxylique est un ester d'un alcool mono-ou plurivalent.

7. Procédé selon la revendication 6, caractérisé en ce que l'ester d'acide carboxylique est un ester d'éthanol, propanol, isopropanol ou glycérol ou un ester cyclique (lactone) ou un ester de bioxyde de carbone.

8. Procédé selon l'une des revendications 1 à 7, caractérisé en ce que l'ester d'acide carboxylique est le lactate d'éthyle.

9. Procédé selon l'une des revendications précédentes, caractérisé en ce que le bicarbonate est du bicarbonate de soude.

10. Procédé selon l'une des revendications précédentes, caractérisé en ce que le procédé est utilisé pour la fabrication d'une solution pour une dialyse péritonéale où la solution contient de plus un agent osmotique.

11. Procédé selon la revendication 10, caractérisé en ce qu'en tant qu'agent osmotique, du glucose est contenu dans la solution.

12. Solution médicinale de perfusion ou de dialyse, avantageusement solution de dialyse péritonéale avec une valeur de pH comprise entre 6,9 et 7,8 avec au moins les composants suivants : bicarbonate, avantageusement bicarbonate de soude, un acide carboxylique physiologiquement supportable et un alcool physiologiquement supportable, où l'acide carboxylique ainsi que l'alcool ont été formés par l'hydrolyse d'un ester d'acide carboxylique.

13. Solution selon la revendication 12, caractérisée en ce qu'il y a au moins 2 mmoles/l, avantageusement 2 à 10 mmoles/l d'ester d'acide carboxylique.

14. Solution selon la revendication 12 ou 13, caractérisée en ce que l'ester d'acide carboxylique provient du groupe acide acétique, acide propanoïque, acide lactique, acide pyruvique, acide 4-hydroxybutyrique, acide succinique, acide maléique, acide fumarique, acide maléïque, acide oxalique, acide glutarique, acide oxoglutarique, acide citrique, acide isocitrique et acide gluconique.

15. Solution selon l'une des revendications 12 à 14, caractérisée en ce que l'ester d'acide carboxylique est le lactate d'éthyle.

16. Solution selon l'une des revendications 12 à 15, caractérisée en ce qu'est contenu de plus une substance ayant un effet osmotique, avantageusement du glucose.

17. Solution selon l'une des revendications 12 à 16, caractérisée en ce qu'elle est obtenue directement avant son utilisation par mélange de deux solutions, dont l'une contient des ions calcium et l'autre du bicarbonate de soude.

18. Solution selon l'une des revendications précédentes, caractérisée en ce qu'elle est obtenue par mélange de deux solutions dont une contient au moins une substance à effet osmotique, avantageusement du glucose, et l'autre du bicarbonate.
